Europäisches Patentamt

European Patent Office

Office européen des brevets

(11) Publication number: **0 335 359**
**A2**

## EUROPEAN PATENT APPLICATION

(21) Application number: **89105508.9**

(22) Date of filing: **29.03.89**

(51) Int. Cl.⁴: **C04B 38/06 , A61L 27/00 , A61K 6/00**

(30) Priority: **31.03.88 JP 79688/88**

(43) Date of publication of application:
**04.10.89 Bulletin 89/40**

(84) Designated Contracting States:
**DE FR GB**

(71) Applicant: **ASAHI KOGAKU KOGYO KABUSHIKI KAISHA**
**36-9, Maeno-cho 2-Chome Itabashi-ku Tokyo(JP)**

(72) Inventor: **Ojima, Satoshi c/o Asahi Kogaku Kogyo K.K.**
**36-9, Maenocho 2-chome**
**Itabashi-ku Tokyo(JP)**
Inventor: **Ogawa, Tetsuro c/o Asahi Kogaku Kogyo K.K.**
**36-9, Maenocho 2-chome**
**Itabashi-ku Tokyo(JP)**

(74) Representative: **Moinas, Michel**
**Moinas & Cie 42, rue Plantamour**
**CH-1201 Genève(CH)**

(54) **Porous ceramic material and production process thereof.**

(57) Porous ceramic materials which are useful as a biomaterial such as an artificial bone, a transcutaneous etc. They are produced from a green compact which is produced by compression molding of a blend of a raw ceramic material and a sublimable binder by removing said binder from the green compact through sublimation thereof. They can exhibit a widely controllable porosity and have a good mechanical strength. A simple and easy process for the production of said porous ceramic materials is also provided.

EP 0 335 359 A2

## POROUS CERAMIC MATERIAL AND PRODUCTION PROCESS THEREOF

Field of the Invention

The present invention relates to a porous ceramic material which is particularly suitable as a biomaterial such as an artificial bone material, a transcutaneous device etc. and to a green compact for the production of said porous ceramic material. The porous ceramic material of the present invention has a widely controllable porosity and a notably increased mechanical strength. The present invention also relates to a process for the production of said porous ceramic material.

Description of Related Art

Porous artificial bone materials such as porous ceramics, for example, calcium phosphate ceramics, have been widely used in the fields of orthopaedics, oral surgery, and the like. The clinically useful bone materials have been produced in accordance with various methods among which there may be mentioned a foaming method in which an aqueous hydrogen peroxide, a surface active agent or other components is(are) added to an apatite slurry and the mixture is then foamed and dried at a temperature of about 80 to 100°C in a constant temperature chamber to obtain a foamed apatite product, an urethane foam method in which an urethane foam is impregnated with a slurry of the raw ceramic material having a good compatibility with human body and then the urethane foam is thermally decomposed to form pores in the resulting bone material, and an acrylic bead method in which a starting material is mixed with beads of the acrylic resin having a diameter of several microns to several hundred microns in either a wet process or a dry process and then the mixture is thermally decomposed to form pores in the resulting bone material. These methods, however, have problems to be solved. For example, the methods which should be performed under wet conditions suffer from very complicated and troublesome operations in preparing, introducing and/or impregnating the slurry and drying the mixture of the starting material and slurry. Further, in the foaming method, it is very difficult to control a porosity of the resultant bone material. Furthermore, although the acrylic bead method may be performed in a dry process, this dry method has drawbacks in that the addition of a much amount of acrylic beads or use of acrylic beads with a remarkably large diameter which is essential to increase a porosity of the porous bone material causes cracks and other defects by application of an isostatic press.

Summary of the Invention

An object of the present invention is to provide a novel porous ceramic material with a widely controllable porosity and a notably increased mechanical strength.

Another object of the present invention is to provide a method for the production of said porous ceramic material which can be easily carried out in the simplest operations, with good results and yields.

Another object of the present invention is to provide a green compact with an increased mechanical strength, from which said porous ceramic material is produced.

According to the present invention, these objects and other objects which will be clarified in the following description of the present invention can be attained by using a specific sublimable solid binder as a pore-forming agent in the production of porous artificial bone materials.

According to the present invention, there is provided a porous ceramic material produced by heating a green compact which is produced by compression molding of a blend of a raw material of said ceramic material and a binder consisting of at least one sublimable material selected from the group consisting of condensed cycloaliphatic hydrocarbons and halogenated aromatic hydrocarbons to remove said binder through sublimation thereof, a porosity of said ceramic material being adjusted to a desired value by controlling an amount of said binder used in said blend.

In addition to the porous ceramic material, according to the present invention, there is also provided a process for the production of a porous ceramic material which comprises the steps of:

mixing a raw ceramic material with a binder consisting of at least one sublimable material selected from the group consisting of condensed cycloaliphatic hydrocarbons and halogenated aromatic hydrocarbons which is added in an amount effective to provide a desired porosity upon sublimation of the binder,

molding the blend to a green compact having a desired configuration,

heating the green compact to over a thermal decomposition temperature of said binder to produce a porous product as a result of sublimation of said binder,

and, if desired, sintering the obtained porous product.

In the porous ceramic material of the present invention, its porosity can be easily and widely controlled by varying an amount of the binder added to the raw ceramic material. Furthermore, the production of the porous ceramic material can be easily attained by using simple operations. In addition to the simple and easy production, it does not cause defects such as cracking in the products and thus ensures increase of the yields of the products with good qualities. No rejected product is produced, insofar as the process of the present invention is applied to the production of the described ceramic material.

The ceramic material of the present invention has excellent characteristics and therefore can be effectively and widely utilized as various biomaterial such as an artificial bone material, an artificial tooth root, a transcutaneous device etc.

## Detailed Description of Embodiments

The present invention can apply to any raw ceramic material. Useful raw ceramic materials are preferably selected from the group consisting of calcium phosphates such as tricalcium phosphate [$Ca_3(PO_4)_2$], apatite, e.g., hydroxyapatite [$Ca_{10}(PO_4)_6(OH)_2$], fluoroapatite and other ceramic materials such as alumina, zirconia. These raw ceramic materials may be used separately or in combination. These raw materials may be preferably used after pulverization to a desired particle size which varies depending on factors such as a specific raw ceramic material, a specific production process of the biomaterial, and the like.

In the ceramic material of the present invention, a binder which consists of at least one sublimable material is used in combination with said raw ceramic material to form a blend. Any sublimable material may be used herein, insofar as it can cause formation of pores in the resultant ceramic material as a result of removal of the same through sublimation thereof. Useful binders include condensed cycloaliphatic hydrocarbons and halogenated aromatic hydrocarbons, for example, adamantane, trimethylene norbornane, p-dichlorobenzene and the like. These binder materials may be used separately or in combination, and more preferably, they may be used as a mixture of the sublimable materials which contains at least adamantane or trimethylene norbonane as a major component thereof. Adamantane and trimethylene norbornane each is preferred, because a sublimed gas produced from these materials has only a low toxicity.

In general, adamantane can be synthesized from trimethylene norbornane with a yield of about 20 to 30%. Several mixtures of adamantane and trimethylene norbornane with varied mixing ratio are available under the trade name of "AISOUR". These mixtures are in the form of granules or wax depending on the content of adamantane and, therefore, preferably used as the binder in the present invention.

The above-listed binder materials are those which have been generally used as a binder or a degreasing accelerator in conventional injection molding and extrusion molding in an amount of about 1 to 3% by weight based on a total weight of the molding materials used. In contrast, for the present invention, the binder materials are used in an amount of about 10 to 80% by weight, more preferably about 20 to 60% by weight, based on a total amount of the blend, i.e., mixture of the ceramic materials and binder materials. The amount of the binder materials added may be widely varied depending upon the desired porosity of the resulting ceramic material. In other words, according to the present invention, a porosity of the ceramic material can be easily controlled by changing a mixing ratio between the solid binder and raw ceramic material used.

The binder materials are in the form of powders, granules or wax. If they are in the form of wax, a large-sized block body of the porous ceramic material will be simply produced in the subsequent molding process, because the blend of the binder material and raw ceramic material tends to easily harden during dry molding.

After mixing the raw ceramic material with the solid binder with the mixing ratio described above, the resulting blend is compression molded into a green compact. Then, the green compact is heated to over a sublimation temperature of said binder to cause sublimation of the same and thus yield a porous ceramic material. The porous ceramic material has, dispersed therein, a multiplicity of pores. In this connection, the inventors found that if a small amount of easily volatilizable solvents such as ethanol and the like is added to either adamantane or trimethylene norbornane or a mixture thereof the mass can be finely divided, thereby producing a blend in which ceramic powders are uniformly distributed. The blend is dried to volatilize the solvents used, and then molded.

In the practice of the present invention, molding can be carried out in a dry process. Since the dry process can be easily operated as compared with a wet process and also can utilize an isostatic press, an adhesion strength between the ceramic powders and therefore a mechanical strength of the finally obtained ceramic material can be increased. As previously described, the dry process has been applied to the prior art acrylic bead method, but it did not provide satisfactory results. In the dry process-based acrylic bead method, beads of the acrylic resin were first shrinked with application of pressure by use of the isostatic press, and then, were undesirably expanded upon reduction of the pressure. Notable expansion of the acrylic beads was particularly observed, if a much amount of the acrylic beads was added to the starting material or the acrylic beads having a relatively large diameter were used, and, in such instances, cracks and other defects were caused in the resulting green compacts.

However, surprisingly, such defects could be avoided in the present invention. This is because the sublimable binder material used in the present invention is in the form of powders, granules or wax and therefore is not expanded, even if a reduced pressure is applied during molding. Due to absence of expansion of the binder material, the present invention can be conducted by using a large amount of said binder material which ensures to produce a porous ceramic material showing a high strength and having a high porosity.

Moreover, the green compact according to the present invention has a high strength enough to operate fabrication such as cutting.

The heating step (namely "degreasing" step) for the green compacts is the step wherein the sublimable binder material used is removed through sublimation thereof. Removal of the binder material means formation of pores in the resulting products. Thus, this step is conducted by heating the green compacts to a temperature higher than a sublimation temperature of the binder material used in the blend. The thus heated products become a porous structure as a result of removal of the binder material therefrom. The fabrication of the porous products can be carried out after the heating step.

The heating step of the green compacts for removal of the binder material need not be a separate step, and can proceed in process of a sintering step described below.

The obtained porous structure or porous ceramic product having dispersed therein pores is further sintered in a furnace, for example. By sintering, the mechanical strength of the porous ceramic product can be notably increased. A wide range of sintering temperatures are useful in the practice of the present invention, and a suitable sintering temperature will be selected depending upon a specific ceramic material used. As an instance, when apatite is used as the ceramic material, the sintering temperature used is generally about 900 to 1400°C, preferably about 1000 to 1300°C. The fabrication of the porous products can also be carried out before or after the sintering.

The porous ceramic materials of the present invention can be advantageously used as biomaterials in the fields of, for example, orthopaedics and oral surgery. For instance, they may be used to treat osseous defects in the human body, and also be used as an implant material, an artificial denture or an artificial joint or other bone parts, a transcutaneous device and the like.

The present invention will be further described with reference to examples. However, it should be understood that the present invention is not limited to these examples and many variations and modifications may be made within the spirit and scope of the invention.

Example 1

A mixture of adamantane and trimethylene norbornane (AISOUR 800 commercially available from Idemitsu Sekiyu Kagaku KK) was mixed with hydroxyapatite powder (products of Asahi Kogaku Kogyo KK; synthetically prepared by dropping phosphoric acid in a slurry of calcium hydroxide in accordance with well-known solution precipitation techniques) in a weight ratio of 2:3. The thus obtained powdered mixture was then molded in a monoaxial pressure molding machine to produce a green compact in the form of a plate. After molding, the green compact was heated at 650°C for 30 hours to remove thermally decomposed components therefrom. A porous product was obtained. This product was cut to obtain a rectangular rod having a size of 10 x 10 x 30 mm. The rod was sintered at 1200°C. A porous sintered product was thus obtained. The tests for the sintered product demonstrate that a porosity of the product is, on an average, about 51.0% and a flexural strength (determined using a three point bending test) is, on an average, about 83.5 kg/cm$^2$.

Example 2

The procedure of the Example 1 was repeated with the exception that the mixture of adamantane and trimethylene norbornane was mixed with the apatite powder in a weight ratio of 3:7. The tests for the sintered product demonstrate that a porosity of the product is, on an average, about 31.0% and a flexural strength thereof is, on an average, about 161 kg/cm$^2$.

Example 3

The procedure of the Example 1 was repeated with the exception that the mixture of adamantane and trimethylene norbornane was mixed with the apatite powder in a weight ratio of 1:1. The tests for the sintered product demonstrate that a porosity of the product is, on an average, about 62.0% and a flexural strength thereof is, on an average, about 57 kg/cm$^2$.

## Example 4

This example is comparative example.

A series of porous apatite products were prepared from the apatite powder used in the Example 1 by using the well-known hydrogen peroxide foaming method. An aqueous hydrogen peroxide in an amount of about several % of the total weight of the starting materials was added to a slurry of the apatite, and then the mixture was foamed and dried at 80 to 100°C in an oven to produce a porous apatite product. The product was sintered at 1200°C. The tests for the sintered apatite products demonstrate that a porosity of the products is in the range of 50 to 55% and a flexural strength thereof is in the range of 45 to 50 kg/cm².

## Claims

1. A porous ceramic material produced by heating a green compact which is produced by compression molding of a blend of raw material of said ceramic material and a binder consisting of at least one sublimable material selected from the group consisting of condensed cycloaliphatic hydrocarbons and halogenated aromatic hydrocarbons to remove said binder through sublimation thereof, a porosity of said ceramic material being adjusted to a desired value by controlling an amount of said binder used in said blend.

2. The ceramic material as claimed in claim 1 wherein said raw ceramic material is used in the form of powders and comprises calcium phosphates such as tricalcium phosphate, apatites, for example, hydroxyapatite, fluoroapatite, and the like, or a mixture thereof.

3. The ceramic material as claimed in claim 1 wherein said binder comprises adamantane, trimethylene norbornane, p-dichlorobenzene or a mixture thereof.

4. The ceramic material as claimed in claim 1 wherein said binder comprises a mixture of the sublimable materials which contains adamantane or trimethylene norbornane as a major component thereof.

5. The ceramic material as claimed in claim 1 wherein said binder comprises a mixture of adamantane and trimethylene norbornane.

6. The ceramic material as claimed in claim 1 wherein said binder is used in an amount of 10 to 80% by weight based on a total weight of said blend.

7. The ceramic material as claimed in claim 1 wherein the resulting porous ceramic material after molding is further sintered at a seleted sintering temperature which depends on a specific ceramic material used.

8. The ceramic material as claimed in claim 1 which is used to treat osseous defects.

9. The ceramic material as claimed in claim 1 which is used as an implant material.

10. The ceramic material as claimed in claim 1 which is used as an artificial denture part in the field of oral surgery.

11. The ceramic material as claimed in claim 1 which is used as an artificial bone part in the field of orthopaedics.

12. A process for the production of a porous ceramic material which comprises the steps of:
mixing a raw ceramic material with a binder consisting of at least one sublimable material selected from the group consisting of condensed cycloaliphatic hydrocarbons and halogenated aromatic hydrocarbons which is added in an amount effective to provide a desired porosity through sublimation of the binder,
molding the blend to a green compact having a desired configuration, and
heating the green compact to over a thermal decomposition temperature of said binder to produce a porous product as a result of sublimation of said binder.

13. The process as claimed in claim 12 wherein fabrication is carried out before or after heating step.

14. The process as claimed in claim 12 or claim 13 which further comprises sintering the resulting porous ceramic material at a selected sintering temperature which depends on a specific ceramic material used.

15. The process as claimed in claim 12 or claim 13 wherein said raw ceramic material is used as powders and comprises calcium phosphates such as tricalcium phosphate, apatites, for example, hydroxyapatite, fluoroapatite, and the like, or a mixture thereof.

16. The process as claimed in claim 12 or claim 13 wherein said binder comprises adamantane, trimethylene norbornane, p-dichlorobenzene or a mixture thereof.

17. The process as claimed in claim 12 or claim 13 where said binder combrises a mixture of the sublimable materials which contains adamantane or trimethylene norbornane as a major component thereof.

18. The process as claimed in claim 12 or claim 13 wherein said binder comprises a mixture of adamantane and trimethylene norbornane.

19. The process as claimed in claim 12 or claim 13 wherein the amount of said binder added to said raw ceramic material is 10 to 80% by weight based on a total weight of the resulting blend.

20. The process as claimed in claim 12 or claim 13 wherein molding is carried out in a dry process using an isostatic press.

21. A green compact produced by compression molding of a blend of a raw ceramic material and a binder consisting of at least one sublimable material selected from the group consisting of condensed cycloaliphatic hydrocarbons and halogenated aromatic hydrocarbons.

22. The green compact as claimed in claim 21 wherein said raw ceramic material is used in the form of powders and comprises calcium phosphates such as tricalcium phosphate, apatites, for example, hydroxyapatite, fluoroapatite, and the like, or a mixture thereof.

23. The green compact as claimed in claim 21 wherein said binder comprises adamantane, trimethylene norbornane, p-dichlorobenzene or a mixture thereof.

24. The green compact as claimed in claim 21 wherein said binder comprises a mixture of the sublimable materials which contains adamantane or trimethylene norbornane as a major component thereof.

25. The green compact as claimed in claim 21 wherein said binder comprises a mixture of adamantane and trimethylene norbornane.

26. The green compact as claimed in claim 21 wherein said binder is used in an amount of 10 to 80% by weight based on a total weight of said blend.